(19) Europäisches Patentamt
European Patent Office
Office européen des brevets

(11) Numéro de publication: **0 520 882 B1**

(12) # FASCICULE DE BREVET EUROPEEN

(45) Date de publication de fascicule du brevet: **12.07.95**

(51) Int. Cl.[6]: **C07D 401/12**, A61K 31/505

(21) Numéro de dépôt: **92401771.8**

(22) Date de dépôt: **24.06.92**

(54) **Dérivés de 2-aminopyrimidine-4-carboxamide, leur préparation et leur application en thérapeutique.**

(30) Priorité: **27.06.91 FR 9107938**
**18.05.92 FR 9206005**

(43) Date de publication de la demande:
**30.12.92 Bulletin 92/53**

(45) Mention de la délivrance du brevet:
**12.07.95 Bulletin 95/28**

(84) Etats contractants désignés:
**AT BE CH DE DK ES FR GB GR IT LI LU MC NL PT SE**

(56) Documents cités:
**EP-A- 0 301 936**

(73) Titulaire: **SYNTHELABO**
**22, Avenue Galilée**
**F-92350 Le Plessis Robinson (FR)**

(72) Inventeur: **George, Pascal**
**19, rue des Ouatre Vents**
**F-78730 St Arnoult en Yvelines (FR)**
Inventeur: **Maloizel, Christian**
**21, rue du Plateau**
**F-92190 Meudon (FR)**
Inventeur: **Marabout, Benoit**
**6, rue Georges Ritz**
**F-91300 Massy (FR)**
Inventeur: **Merly, Jean-Pierre**
**11, avenue Jules Guesde**
**F-92330 Sceaux (FR)**

(74) Mandataire: **Ludwig, Jacques et al**
**SYNTHELABO,**
**Service Brevets,**
**B.P. 72**
**F-92352 Le Plessis Robinson Cédex (FR)**

**EP 0 520 882 B1**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention a pour objet des dérivés de 2-aminopyrimidine-4-carboxamide, leur préparation et leur application en thérapeutique.

Les composés de l'invention répondent à la formule générale (I)

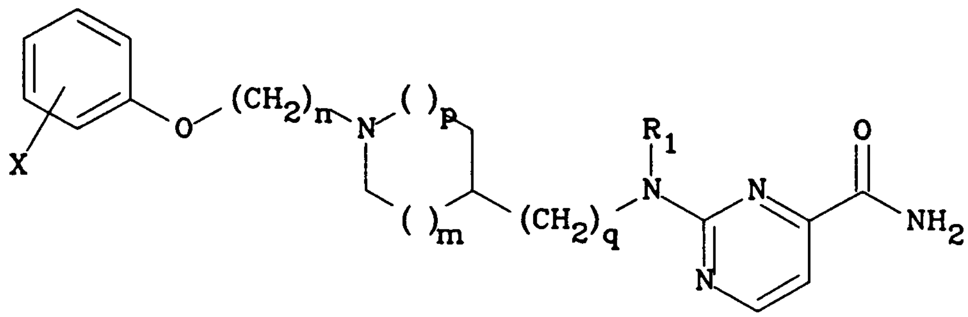

(I)

dans laquelle

- X représente un ou plusieurs substituants choisis parmi les atomes d'hydrogène, de fluor et de chlore et les groupes méthyle, 1-méthyléthyle et méthoxy,
- n représente le nombre 2 ou 3,
- m représente le nombre 1, auquel cas p représente le nombre 1, ou bien
- m représente le nombre 0, auquel cas p représente le nombre 2,
- q représente le nombre 0 ou 1, et
- $R_1$ représente un atome d'hydrogène.

Les composés de l'invention peuvent se présenter à l'état de bases ou de sels d'addition à des acides, ainsi que sous forme d'énantiomères purs ou de mélanges d'énantiomères, par exemple racémiques.

Conformément à l'invention, on peut préparer les composés de formule générale (I) selon l'un des deux procédés illustrés par les schémas 1 et 2 qui suivent.

Le schéma 1 concerne seulement les composés de formule générale (I) dans laquelle m = p = 1 et q = 0, ce qui correspond à la formule générale (I').

Selon ce schéma, on fait réagir la pipéridine substituée de formule (II') avec un halogénure de phénoxyalkyle de formule

**Schéma 1**

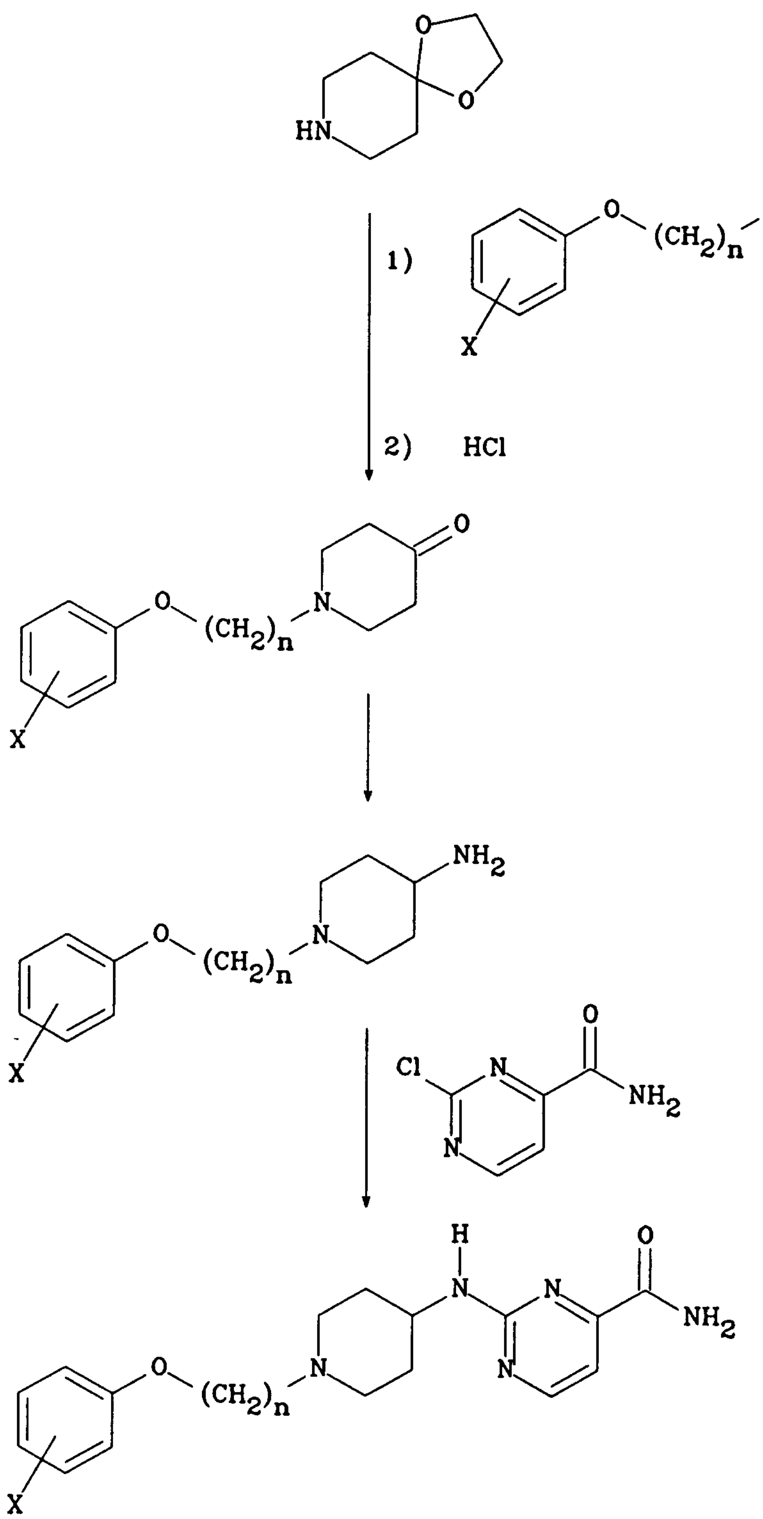


(II')

(III)

(IV')

(V')

(VI)

(I')

générale (III), dans laquelle X et n sont tels que définis ci-dessus et Y représente un atome de chlore ou de brome, dans la butan-2-one en présence d'une base minérale, par exemple le carbonate de potassium, à une température de 80 °C. Ensuite on transforme l'acétal obtenu en cétone de formule générale (IV') par hydrolyse, par exemple avec de l'acide chlorhydrique.

Lorsque tous les substituants X sont différents d'un atome d'halogène, on peut transformer la cétone de

formule générale (IV′) en amine de formule générale (V′) par réaction avec l'ammoniac dans un alcool aliphatique, par exemple le méthanol, en présence de charbon palladié à 10 % sous atmosphère d'hydrogène.
Lorsque l'un des substituants X est un atome d'halogène, on peut faire réagir la cétone de formule générale (IV′) avec la méthoxyamine dans de l'éthanol aqueux, puis on réduit l'oxime obtenue par un hydrure alcalin, par exemple l'hydrure de lithium et d'aluminium, dans un solvant éthéré tel que le tétrahydrofurane, pour obtenir l'amine de formule générale (V′).

On obtient le dérivé de 2-aminopyrimidine-4-carboxamide de formule générale (I′) en faisant réagir l'amine de formule (V′) avec le 2-chloropyrimidine-4-carboxamide de formule (VI), dans un solvant aprotique, par exemple le *N,N*-diméthylformamide, en présence d'une base minérale, par exemple le carbonate de potassium, à une température de 20 à 40 °C.

La pipéridine substituée de formule (II′), ou 1,4-dioxa-8-azaspiro[4.5]décane, est disponible dans le commerce.

Les halogénures de phénoxyalkyle de formule générale (III) peuvent être préparés par des méthodes analogues à celles décrites dans *J. Pharm. Sci.*, 1984, **73/9**, 1241-1244, ou dans *Synthesis*, 1990, 1069-1071.

Le 2-chloropyrimidine-4-carboxamide de formule (VI) peut être préparé à partir de 2-chloropyrimidine-4-carbonitrile par traitement à l'acide chlorhydrique gazeux dans l'acide formique, ledit nitrile étant lui-même préparé selon la méthode décrite dans *J. Het. Chem.*, 1964, **1**, 130-133.

Selon le schéma 2 ci-dessous, on fait d'abord réagir une amine de formule générale (II), dans laquelle m, p, q et $R_1$ sont tels que définis ci-dessus et R représente un groupement protecteur de l'amine, par exemple un groupe benzyloxycarbonyle ou t.-butoxycarbonyle, avec un halogénure de phénoxyalkyle de formule générale (III), dans laquelle X et n sont tels que définis ci-dessus et Y représente un atome de chlore ou de brome, dans un solvant aprotique, par exemple le *N,N*-diméthylformamide, en présence d'une base, par exemple minérale telle que le carbonate de potassium, à une température de 40 à 100 °C.

On obtient une diamine de formule générale (IV), que l'on déprotège ensuite, selon la nature du groupement protecteur R, par un méthode analogue à celles décrites dans la littérature, par exemple par hydrogénation en présence de charbon palladié (dans le cas d'un groupe benzyloxycarbonyle) ou par réaction avec l'acide trifluoroacétique dans le dichlorométhane (dans le cas d'un groupe t.-butoxycarbonyle).

On obtient une diamine de formule générale (V), que l'on fait finalement réagir avec le 2-chloropyrimidine-4-carboxamide de formule (VI) dans un solvant aprotique, par exemple le *N,N*-diméthylformamide, en présence d'une base, par exemple minérale telle que le carbonate de potassium, à une température de 20 à 60 °C.

Les diamines protégées de formule générale (II), dans laquelle q représente le nombre 1, peuvent être préparées par une méthode analogue à celles décrites pour la synthèse du pipéridine-4-carbamate de t.-butyle (q = 0) dans les demandes de brevets DE-2831431, EP-410278 et EP-417698.

Les halogénures de phénoxyalkyle de formule générale (III) et le 2-chloropyrimidine-4-carboxamide de formule (VI) peuvent être préparés comme décrit à propos du schéma 1.

Schéma 2

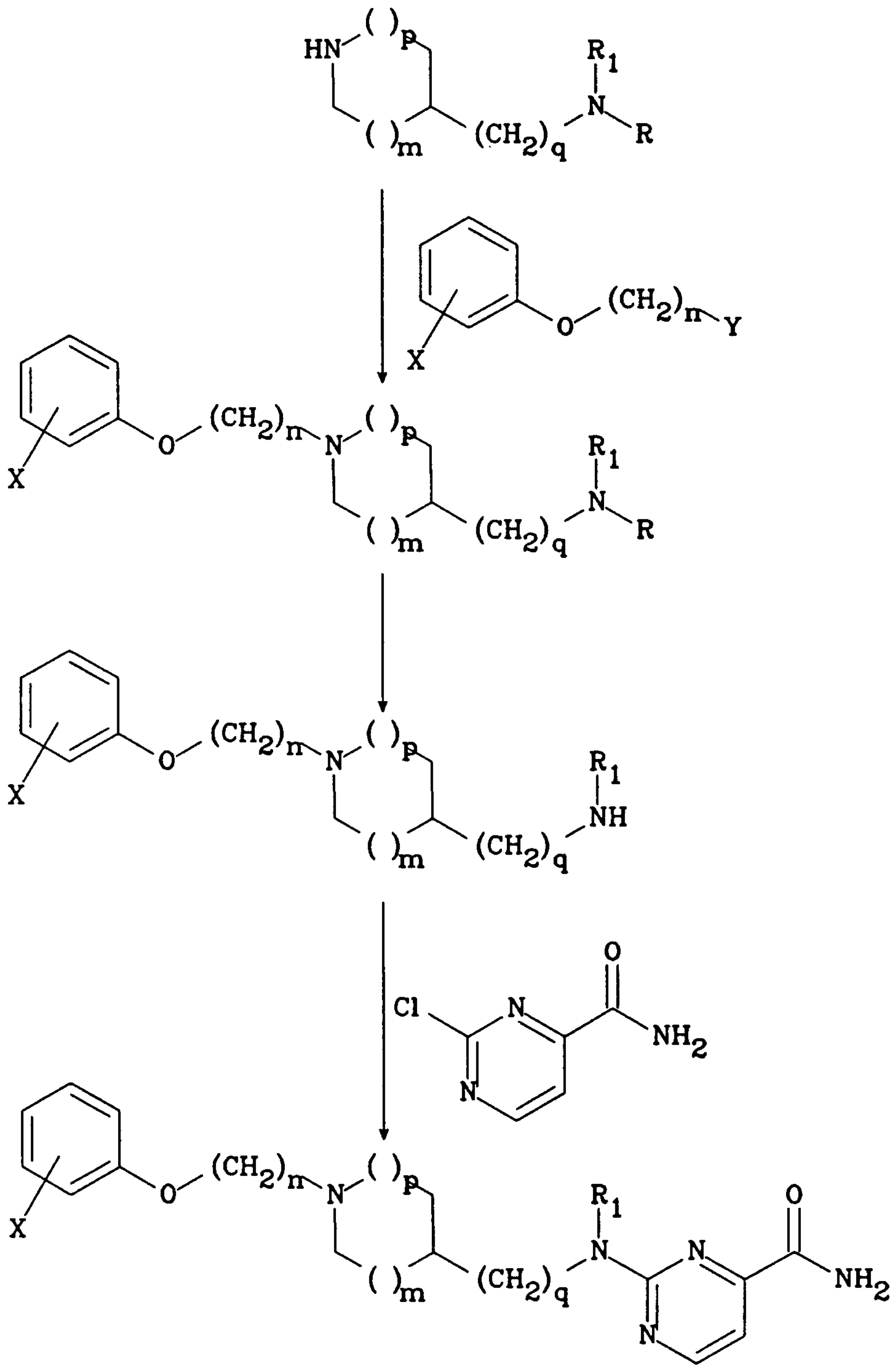

(II)

(III)

(IV)

(V)

(VI)

(I).

Les exemples suivants illustrent en détail la préparation de quelques composés selon l'invention. Les microanalyses élémentaires et les spectres IR et RMN confirment les structures des produits obtenus. Les numéros entre parenthèses dans les titres correspondent à ceux du tableau donné plus loin.

Exemple 1 (Composé N° 1)

2-[1-[2-(2-méthoxyphénoxy)éthyl]pipéridin-4-ylamino]pyrimidine-4-carboxamide, chlorhydrate.

1.1. 8-[2-(2-méthoxyphénoxy)éthyl]-1,4-dioxa-8-azaspiro[4.5]décane.

Dans un ballon de 1 litre on introduit 32,2 g (0,139 mole) de bromure de 2-(2-méthoxyphénoxy)éthyle, 19,95 g (0,139 mole) de 28,8 g (0,208 mole) de carbonate de potassium et 322 ml de butan-2-one. On chauffe et agite le mélange à la température du reflux du solvant pendant 7 heures. On refroidit le mélange, on élimine l'insoluble par filtration et on concentre le filtrat sous pression réduite pour obtenir 40,8 g d'huile.

1.2. 1-[2-(2-méthoxyphénoxy)éthyl]pipéridin-4-one.

On dissout 40,8 g (0,139 mole) de 8-[2-(2-méthoxyphénoxy)éthyl]-1,4-dioxa-8-azaspiro[4.5]décane dans 408 ml d'acide acétique et 40,8 ml d'acide chlorhydrique concentré. On porte le mélange à la température du reflux pendant 30 minutes. On concentre le mélange sous pression réduite puis reprend le résidu avec un peu d'eau et de dichlorométhane. On alcalinise la solution avec de l'ammoniaque concentrée, on extrait au dichlorométhane puis sèche la phase organique sur sulfate de magnésium et évapore le solvant sous pression réduite. On recristallise le solide obtenu dans du propan-2-ol pour obtenir 25,4 g de produit.
Point de fusion : 73-75 °C.

1.3. 1-[2-(2-méthoxyphénoxy)éthyl]pipéridin-4-amine, chlorhydrate.

Dans un flacon de 500 ml d'un appareil de Parr, on introduit 5,4 g (0,0216 mole) de 1-[2-(2-méthoxyphénoxy)éthyl]pipéridin-4-oneet 200 ml de méthanol. On refroidit le mélange par un bain de glace et on sature la solution avec de l'ammoniac puis, on ajoute 0,5 g de palladium sur charbon à 10 %.
On agite le mélange pendant 5 heures sous atmosphère d'hydrogène à pression ambiante, puis on filtre et on concentre sous pression réduite.
On prépare le chlorhydrate du composé que l'on fait recristalliser dans un mélange propan-2-ol/acétate d'éthyle. On obtient 2,2 g de composé.
Point de fusion : 128-131 °C.

1.4. 2-[1-[2-(2-méthoxyphénoxy)éthyl]pipéridin-4-ylamino]pyrimidine-4-carboxamide, chlorhydrate.

Dans un ballon de 100 ml on introduit 2,1 g (0,0073 mole) de 1-[2-(2-méthoxyphénoxy)éthyl]pipéridin-4-amine, 1,15 g (0,0073 mole) de 2-chloropyrimidine-4-carboxamide et 3 g (0,0219 mole) de carbonate de potassium en solution dans 42 ml de *N,N*-diméthylformamide.
On agite le mélange pendant 48 heures à la température ambiante, on le verse dans de l'eau, puis on extrait trois fois avec de l'acétate d'éthyle. On lave la phase organique une fois à l'eau, on la sèche sur sulfate de sodium, on la filtre et on concentre le filtrat sous pression réduite. On recristallise la base dans du propan-2-ol pour obtenir 0,7 g de base.
A la base en solution dans un mélange de dichlorométhane et de propan-2-ol, on ajoute 19 ml d'une solution d'acide chlorhydrique 0,1N dans du propan-2-ol. On évapore le solvant sous pression résuite et on fait recristalliser le chlorhydrate dans du propan-2-ol. On obtient 0,26 g de composé.
Point de fusion : 194-196 °C.

<u>Exemple 2</u> (Composé N° 2)

2-[1-[2-(4-fluorophénoxy)éthyl]pipéridin-4-ylamino]pyrimidine-4-carboxamide, chlorhydrate.

2.1. 8-[2-(4-fluorophénoxy)éthyl]-1,4-dioxa-8-azaspiro[4.5]décane.

On introduit 21,91 g (0,1 mole) de bromure de 2-(4-fluorophénoxy)éthyle, 14,32 g (0,1 mole) de 1,4-dioxa-8-azaspiro[4.5]décane et 20,73 g (0,15 mole) de carbonate de potassium dans 250 ml de butan-2-one, et on chauffe le mélange à ébullition pendant 7 heures.
On refroidit le mélange à la température ambiante, on filtre, lave l'insoluble avec 200 ml d'éther et on évapore les solvants sous pression réduite. On obtient 29 g d'une huile jaune que l'on utilise telle quelle dans l'étape suivante.

2.2. 1-[2-(4-fluorophénoxy)éthyl]pipéridin-4-one.

A une solution de 28,13 g (0,1 mole) de 8-[2-(4-fluorophénoxy)éthyl]-1,4-dioxa-8-azaspiro[4.5]décane dans 300 ml d'acide acétique, on ajoute 30 ml d'acide chlorhydrique concentré. On chauffe le mélange pendant 1 heure à ébullition, on l'évapore sous pression réduite, on ajoute au résidu 150 ml de soude 1N et on extrait au dichlorométhane. On sèche la phase organique sur sulfate de magnésium, on filtre et évapore le solvant sous pression réduite. On obtient 24,9 g de produit que l'on utilise tel quel dans l'étape suivante.

2.3. 1-[2-(4-fluorophénoxy)éthyl]pipéridin-4-amine.

On place 20,15 g (0,085 mole) de 1-[2-(4-fluorophénoxy)éthyl]pipéridin-4-one, 10,65 g (0,1275 mole) de chlorhydrate de méthoxyamine, 11,51 g (0,140 mole) d'acétate de sodium dans 300 ml d'éthanol et 80 ml d'eau, et on chauffe le mélange pendant 2 heures.
On évapore l'éthanol, ajoute 100 ml d'eau et on extrait au dichlorométhane. On sèche la phase organique sur sulfate de magnésium, on filtre et évapore sous pression réduite. Dans 150 ml de tétrahydrofurane, on met en suspension 4,84 g (0,127 mole) d'hydrure de lithium et d'aluminium, puis on ajoute, goutte à goutte, le produit brut obtenu précédemment. On chauffe le mélange à la température de reflux pendant 18 heures. On hydrolyse l'excès d'hydrure avec 5 ml de soude, on filtre sur sulfate de magnésium, et on évapore le solvant sous pression réduite. On reprend le résidu avec de l'eau, on ajoute 100 ml de soude 2N et on extrait au dichlorométhane. On sèche la phase organique sur sulfate de magnésium, on filtre et évapore le solvant. On obtient le composé sous forme de base.
On prépare le chlorhydrate en ajoutant une solution d'acide chlorhydrique 0,1N dans 800 ml de propan-2-ol. On obtient 9,93 g de composé.
Point de fusion : 159-162 °C.

2.4. 2-[1-[2-(4-fluorophénoxy)éthyl]pipéridin-4-ylamino]pyrimidine-4-carboxamide, chlorhydrate.

Sous argon, on met en suspension 5,5 g (0,02 mole) de 1-[2-(4-fluorophénoxy)éthyl]pipéridin-4-amine, 3,15 g (0,02 mole) de 2-chloropyrimidine-4-carboxamide, 6,91 g (0,05 mole) de carbonate de potassium et 0,4 g d'iodure de sodium dans 40 ml de *N,N*-diméthylformamide.
On agite le mélange à la température ambiante pendant 17 heures puis à 40-45 °C pendant 8 heures. On refroidit le mélange à la température ambiante, on le verse sur 150 ml d'eau et on l'extrait avec de l'acétate d'éthyle. On lave la phase organique à l'eau, la sèche sur sulfate de magnésium, on filtre et évapore le solvant sous pression réduite. Après chromatographie sur silice (éluant : dichlorométhane/méthanol 97,5/2,5 à 90/10), on obtient 4,95 g de base.
On en prépare le chlorhydrate par réaction avec une solution 0,1N d'acide chlorhydrique dans du propan-2-ol. Après recristallisation dans de l'éthanol on obtient 4,1 g de composé.
Point de fusion : 202-205 °C.

Exemple 3 (Composé N °8)

2-[[[1-[2-(4-fluorophénoxy)éthyl]pipéridin-4-yl]méthyl]amino]pyrimidine-4-carboxamide, fumarate.

3.1. [[1-[2-(4-fluorophénoxy)éthyl]pipéridin-4-yl]méthyl]carbamate de 1,1-diméthyléthyle.

Dans un ballon tricol de 250 ml on introduit 6,8 g (0,031 mole) de bromure de 2-(4-fluorophénoxy)-éthyle, 6,0 g (0,028 mole) de [(pipéridin-4-yl)méthyl]carbamate de 1,1-diméthyléthyle, 5,8 g de carbonate de potassium et 60 ml de *N,N*-diméthylformamide, et on chauffe le mélange entre 90 et 100 °C pendant 8 heures sous atmosphère d'argon.
On le refroidit à température ambiante, on le verse sur 250 ml d'eau, et on l'extrait avec de l'acétate d'éthyle. On lave la phase organique à l'eau, on la sèche sur sulfate de magnésium, on la filtre et on évapore le solvant sous pression réduite.
Après purification par chromatographie sur colonne de gel de silice (éluant : dichlorométhane/méthanol 98/2 à 92/8) on isole 9 g de produit huileux qu'on utilise tel quel dans l'étape suivante.

3.2. 1-[2-(4-fluorophénoxy)éthyl]pipéridin-4-méthanamine.

Dans un ballon tricol de 500 ml on introduit 8,8 g (0,025 mole) de [[1-[2-(4-fluorophénoxy)éthyl]-pipéridin-4-yl]méthyl]carbamate de 1,1-diméthyléthyle, 90 ml de dichlorométhane et 90 ml d'acide trifluoroacétique, et on chauffe le mélange à 40 °C pendant 4,5 heures.
On le concentre sous pression réduite, on ajoute au résidu brut 200 ml de solution de soude 1N, et on extrait avec du dichlorométhane.
On lave la phase organique à l'eau, on la sèche sur sulfate de magnésium, on la filtre et on évapore le solvant sous pression réduite.
On obtient 6,2 g d'huile jaune qu'on utilise telle quelle dans l'étape suivante.

3.3. 2-[[[1-[2-(4-fluorophénoxy)éthyl]pipéridin-4-yl]méthyl]amino]pyrimidine-4-carboxamide, fumarate.

Sous atmosphère d'argon on prépare une suspension de 3,1 g (0,0123 mole) de 1-[2-(4-fluorophénoxy)-éthyl]pipéridin-4-méthanamine, 2,0 g (0,0127 mole) de 2-chloropyrimidine-4-carboxamide, 2,55 g (0,0185 mole) de carbonate de potassium, 0,3 g d'iodure de sodium et 65 ml de *N,N*-diméthylformamide, et on chauffe le mélange à 60 °C pendant 7,5 heures.
On le refroidit à température ambiante, on le verse sur 150 ml d'eau, on extrait avec de l'acétate d'éthyle, on lave la phase organique à l'eau, on la sèche sur sulfate de magnésium, on la filtre et on évapore le solvant sous pression réduite. On reprend le résidu avec de l'éther diéthylique, et on obtient 3,5 g de base.
On prépare le fumarate par addition de 1,07 g (0,009 mole) d'acide fumarique, en solution dans 100 ml d'éthanol, à 3,45 g (0,009 mole) de base, en solution dans 200 ml d'éthanol. On évapore le solvant sous pression réduite et on recristallise le résidu dans le propan-2-ol.
On obtient finalement 2,9 g de fumarate acide.
Point de fusion : 167-169,5 °C.

<u>Exemple 4</u> (Composé N° 10)

2-[[[1-[2-(2-méthoxyphénoxy)éthyl]pipéridin-3-yl]méthyl]amino]pyrimidine-4-carboxamide, fumarate.

4.1. [[1-[2-(2-méthoxyphénoxy)éthyl]pipéridin-3-yl]méthyl]carbamate de 1,1-diméthyléthyle.

Dans un ballon de Keller de 150 ml on introduit 4,62 g (0,02 mole) de bromure de 2-(2-méthoxyphénoxy)éthyle, 4,29 g (0,02 mole) de [(pipéridin-3-yl)méthyl]carbamate de 1,1-diméthyléthyle, 3,32 g (0,024 mole) de carbonate de potassium et 40 ml de *N,N*-diméthylformamide, et on chauffe le mélange entre 80 et 90 °C pendant 6 heures sous atmosphère d'argon.
On le refroidit à température ambiante, on le verse sur 500 ml d'eau et on extrait avec de l'acétate d'éthyle.
On lave la phase organique à l'eau, on la sèche sur sulfate de magnésium, on la filtre et on évapore le solvant sous pression réduite.
On reprend le résidu avec de l'éther de pétrole, et on obtient 6,8 g de solide qu'on utilise tel quel dans l'étape suivante.
Point de fusion : 78-79 °C.

4.2. 1-[2-(2-méthoxyphénoxy)éthyl]pipéridine-3-méthanamine.

Dans un ballon de 250 ml on introduit 6,8 g (0,0186 mole) de [[1-[2-(2-méthoxyphénoxy)éthyl]pipéridin-3-yl]méthyl]carbamate de 1,1-diméthyléthyle, 55 ml de dichlorométhane et 55 ml d'acide trifluoroacétique, et on chauffe le mélange à 40 °C pendant 4,5 heures.
On le concentre sous pression réduite, on ajoute au résidu brut 200 ml de solution de soude 1N, et on extrait avec du dichlorométhane.
On lave la phase organique à l'eau, on la sèche sur sulfate de magnésium, on la filtre et on évapore le solvant sous pression réduite.
On obtient 4,76 g d'huile jaunâtre qu'on utilise telle quelle dans l'étape suivante.

4.3. 2-[[[1-[2-(2-méthoxyphénoxy)éthyl]pipéridin-3-yl]méthyl]amino]pyrimidine-4-carboxamide, fumarate.

Sous atmosphère d'argon on prépare une suspension de 4,76 g (0,018 mole) de 1-[2-(2-méthoxyphénoxy)éthyl]pipéridine-3-méthanamine, 2,84 g (0,018 mole) de 2-chloropyrimidine-4-carboxamide, 3 g

(0,0216 mole) de carbonate de potassium, 0,3 g d'iodure de sodium et 36 ml de *N,N*-diméthylformamide, et on agite le mélange à température ambiante pendant 24 heures.
On le verse sur 500 ml d'eau, on extrait avec de l'acétate d'éthyle, on lave la phase organique à l'eau, on la sèche sur sulfate de magnésium, on la filtre et on évapore le solvant sous pression réduite.
Après purification du résidu par chromatographie sur colonne de gel de silice (éluant : dichlorométhane/méthanol 98/2 à 90/10) on isole finalement 4,4 g de base.
On prépare le fumarate par addition de 1,33 g (0,0114 mole) d'acide fumarique, en solution dans 100 ml d'éthanol, à 4,4 g (0,0114 mole) de base, en solution dans 100 ml d'éthanol. On évapore le solvant sous pression réduite et on recristallise le résidu dans un mélange de propan-2-ol et d'éthanol.
On obtient finalement 2,65 g de fumarate acide.
Point de fusion : 154-156 °C.

Exemple 5 (Composé N° 4)

2-[1-[2-[5-méthyl-2-(1-méthyléthyl)phénoxy]éthyl]pipéridin-4-ylamino]pyrimidine-4-carboxamide, fumarate.

5.1. [1-[2-[5-méthyl-2-(1-méthyléthyl)phénoxy]éthyl]pipéridin-4-yl]carbamate de 1,1-diméthyléthyle.

Dans un ballon tricol de 250 ml on introduit 5,48 g (0,0231 mole) de bromure de 2-[5-méthyl-2-(1-méthyléthyl)phénoxy]éthyle, 3,87 g (0,0193 mole) de (pipéridin-4-yl)carbamate 1,1-diméthyléthyle, 4,0 g (0,029 mole) de carbonate de potassium et 40 ml de *N,N*-diméthylformamide, et on chauffe le mélange entre 90 et 100 °C pendant 8 heures sous atmosphère d'argon.
On le refroidit à température ambiante, on le verse sur 200 ml d'eau et on l'extrait avec de l'acétate d'éthyle.
On lave la phase organique à l'eau, on la sèche sur sulfate de magnésium, on la filtre et on évapore le solvant sous pression réduite. On purifie le résidu par chromatographie sur colonne de gel de silice en éluant avec un mélange dichlorométhane/méthanol de 98/2 à 95/5. On obtient 7,1 g de solide amorphe qu'on utilise tel quel dans l'étape suivante.

5.2. 1-[2-[5-méthyl-2-(1-méthyléthyl)phénoxy]éthyl]pipéridin-4-amine.

Dans un ballon tricol de 250 ml on introduit 7,0 g (0,0186 mole) de [1-[2-[5-méthyl-2-(1-méthyléthyl)-phénoxy]éthyl]pipéridin-4-yl]carbamate de 1,1-diméthyléthyle, 70 ml de dichlorométhane et 70 ml d'acide trifluoroacétique, et on chauffe le mélange à 40 °C pendant 4 heures.
On le dilue avec 150 ml de dichlorométhane, on le refroidit à 0 °C, et on y fait passer un courant d'ammoniac gazeux. On élimine l'insoluble par filtration, on évapore le solvant sous pression réduite, on reprend le résidu avec du dichlorométhane, on sèche la solution sur sulfate de magnésium, on la filtre et on l'évapore sous pression réduite.
On obtient 5,05 g d'huile orangée qu'on utilise telle quelle dans l'étape suivante.

5.3. 2-[1-[2-[5-méthyl-2-(1-méthyléthyl)phénoxy]éthyl]pipéridin-4-ylamino]pyrimidine-4-carboxamide, fumarate.

Sous atmosphère d'argon on prépare une suspension de 3,4 g (0,0123 mole) de 1-[2-[5-méthyl-2-(1-méthyléthyl)phénoxy]éthyl]pipéridin-4-amine, 2,0 g (0,0127 mole) de 2-chloropyrimidine-4-carboxamide, 2,55 g (0,0185 mole) de carbonate de potassium, 0,3 g d'iodure de sodium et 65 ml de *N,N*-diméthylformamide, et on la chauffe à 60 °C pendant 7,5 heures.
On refroidit le mélange à température ambiante, on le verse sur 150 ml d'eau, on l'extrait avec de l'acétate d'éthyle, on lave la phase organique à l'eau, on la sèche sur sulfate de magnésium, on la filtre et on évapore le solvant sous pression réduite. On reprend le résidu avec de l'éther diéthylique, ce qui fournit 3,4 g de base qu'on recristallise dans le toluène pour obtenir 2,6 g de solide blanc.
Point de fusion : 157,5-160 °C.
Pour en préparer le fumarate on la dissout dans 150 ml d'éthanol et on ajoute 0,76 g (0,0064 mole) d'acide fumarique en solution dans 100 ml d'éthanol, on évapore le solvant sous pression réduite et on recristallise le résidu dans le méthanol.
On obtient finalement 3,6 g de fumarate acide.
Point de fusion : 241-244 °C.

Exemple 6 (Composé N° 5)

2-[1-[3-(5-fluoro-2-méthoxyphénoxy)propyl]pipéridin-4-ylamino]pyrimidine-4-carboxamide, fumarate.

6.1. [1-[3-(5-fluoro-2-méthoxyphénoxy)propyl]pipéridin-4-yl]carbamate de 1,1-diméthyléthyle.

Dans un ballon tricol de 250 ml on introduit 6,35 g (0,0241 mole) de bromure de 3-(5-fluoro-2-méthoxyphénoxy)propyle, 4,37 g (0,0218 mole) de (pipéridin-4-yl)carbamate de 1,1-diméthyléthyle, 4,5 g de carbonate de potassium et 45 ml de *N,N*-diméthylformamide, et on chauffe le mélange entre 90 et 100 °C pendant 8,5 heures sous atmosphère d'argon.
On le refroidit à température ambiante, on le verse sur 250 ml d'eau et on l'extrait avec de l'acétate d'éthyle.
On lave la phase organique à l'eau, on la sèche sur sulfate de magnésium, on la filtre et on évapore le solvant sous pression réduite. On purifie le résidu par chromatographie sur colonne de gel de silice en éluant avec un mélange dichlorométhane/méthanol de 97/3 à 92/8. On obtient 7,3 g de solide amorphe qu'on utilise tel quel dans l'étape suivante.

6.2. 1-[3-(5-fluoro-2-méthoxyphénoxy)propyl]pipéridin-4-amine.

Dans un ballon tricol de 250 ml on introduit 5,7 g (0,0149 mole) de [1-[3-(5-fluoro-2-méthoxyphénoxy)-propyl]pipéridin-4-yl]carbamate de 1,1-diméthyléthyle, 60 ml de dichlorométhane et 60 ml d'acide trifluoroacétique, et on chauffe le mélange à 40 °C pendant 4 heures.
On le dilue avec 200 ml de dichlorométhane, on le refroidit à 0 °C et on y fait passer un courant d'ammoniac gazeux. On élimine l'insoluble par filtration, on évapore le solvant sous pression réduite, on reprend le résidu avec du dichlorométhane, on sèche la solution sur sulfate de magnésium, on la filtre et on évapore le solvant sous pression réduite.
On obtient 4,1 g d'huile orangée qu'on utilise telle quelle dans l'étape suivante.

6.3. 2-[1-[3-(5-fluoro-2-méthoxyphénoxy)propyl]pipéridin-4-ylamino]pyrimidine-4-carboxamide, fumarate.

Sous atmosphère d'argon on prépare une suspension de 3,5 g (0,0123 mole) de 1-[3-(5-fluoro-2-méthoxyphénoxy)propyl]pipéridin-4-amine, 2,0 g (0,0127 mole) de 2-chloropyrimidine-4-carboxamide, 2,55 g (0,0158 mole) de carbonate de potassium, 0,3 g d'iodure de sodium et 65 ml de *N,N*-diméthylformamide, et on la chauffe à 60 °C pendant 7 heures.
On refroidit le mélange à température ambiante, on le verse sur 150 ml d'eau, on l'extrait avec de l'acétate d'éthyle, on lave la phase organique à l'eau, on la sèche sur sulfate de magnésium, on la filtre et on évapore le solvant sous pression réduite. On reprend le résidu avec de l'éther diéthylique, et on obtient 3,3 g de base qu'on recristallise dans le toluène pour obtenir 2,55 g de solide.
On dissout ce dernier dans 150 ml d'éthanol, on ajoute 0,74 g (0,00632 mole) d'acide fumarique en solution dans 80 ml d'éthanol, on évapore le solvant sous pression réduite et on recristallise le résidu dans le méthanol.
On obtient finalement 2,5 g de fumarate acide.
Point de fusion : 244,5-247 °C.

Le tableau suivant illustre les structures chimiques et les propriétés physiques de quelques composés selon l'invention.

**Tableau**

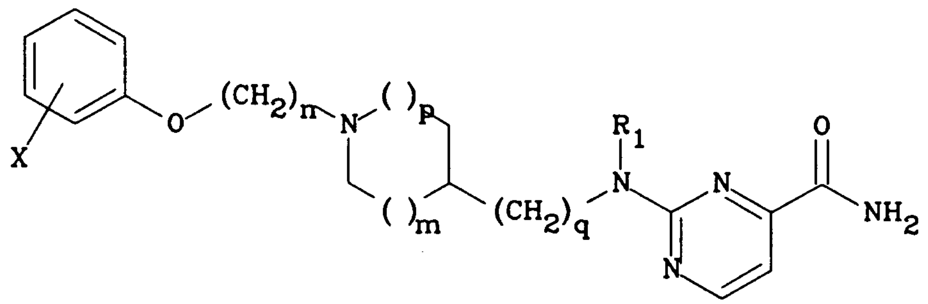

(I)

| N° | X | n | m,p | q | $R_1$ | Sel | F (°C) |
|---|---|---|---|---|---|---|---|
| 1 | 2-$OCH_3$ | 2 | 1,1 | 0 | H | HCl | 194-196 |
| 2 | 4-F | 2 | 1,1 | 0 | H | HCl | 202-205 |
| 3 | 2-$OCH_3$, 5-F | 2 | 1,1 | 0 | H | fum | 211-213,5 |
| 4 | 2-i$C_3H_7$, 5-$CH_3$ | 2 | 1,1 | 0 | H | fum | 241-244 |
| 5 | 2-$OCH_3$, 5-F | 3 | 1,1 | 0 | H | fum | 244,5-247 |
| 6 | 4-F | 3 | 1,1 | 0 | H | fum | 239-241 |
| 7 | H | 2 | 0,2 | 1 | H | fum | 167,5-170 |
| 8 | 4-F | 2 | 1,1 | 1 | H | fum | 167-169,5 |
| 9 | 4-F | 2 | 0,2 | 1 | H | ⅔fum | 170-173 |
| 10 | 2-$OCH_3$ | 2 | 0,2 | 1 | H | fum | 154-156 |
| 11 | 2-$OCH_3$, 5-Cl | 2 | 1,1 | 1 | H | fum | 172,5-175 |

Note : dans la colonne "X", "i$C_3H_7$" désigne un groupe 1-méthyléthyle ; dans la colonne "Sel", "HCl" désigne un chlorhydrate, "fum." désigne un fumarate acide (1 mole de base pour 1 mole de diacide) et "⅔fum." désigne un fumarate composé de 2 moles de base et de 3 moles de diacide.

Les composés de l'invention ont fait l'objet d'études quant à leur activité antagoniste des récepteurs $\alpha_1$-adrénergiques au niveau du bas appareil urinaire.

Leur activité *in vitro* a été étudiée sur l'urètre isolé de lapin.

On prépare des anneaux d'urètre de lapin adulte selon la méthode de Ueda et al., *Eur. J. Pharmacol.*, (1984), **103**, 249-254, puis, après sensibilisation à la noradrénaline, on détermine la courbe concentration-réponse à la phényléphrine, en absence et en présence de composé à étudier.
On évalue la puissance de l'antagonisme $\alpha_1$-adrénergique de chaque composé par calcul du $pA_2$, antilogarithme de la concentration molaire d'antagoniste en présence de laquelle la concentration d'agoniste doit être doublée pour engendrer le même effet qu'en son absence.
Les $pA_2$ des composés sont de l'ordre de 5,5 à 9.

L'activité *in vivo* des composés de l'invention a été étudiée quant à leur effet sur l'hypertonie urétrale engendrée par la stimulation des fibres sympathiques du nerf hypogastrique chez le chat anesthésié.
On anesthésie des chats mâles adultes au pentobarbital sodique, et on les prépare selon la méthode de Theobald, *J. Auton. Pharmac.*, (1983), **3**, 235-239, afin d'obtenir une hypertonie urétrale par stimulation des fibres sympathiques du nerf hypogastrique. On note les réponses contractiles de l'urètre à la stimulation électrique du nerf hypogastrique avant et après administration intraveineuse des composés à étudier, à doses cumulatives de 1 à 1000 µg/kg.
On évalue la puissance de l'antagonisme $\alpha_1$-adrénergique de chaque composé par calcul de la $DI_{50}$, dose qui inhibe de 50% l'hypertonie urétrale.
Les $DI_{50}$ des composés de l'invention sont de l'ordre de 0,01 à 1 mg/kg.

Les résultats des essais montrent que les composés de l'invention montrent, *in vitro*, une activité antagoniste des récepteurs $\alpha_1$-adrénergiques des muscles lisses du bas appareil urinaire (urètre) stimulés par un agoniste $\alpha_1$-adrénergique (phényléphrine). *In vivo*, ils inhibent l'hypertonie urétrale engendrée par la stimulation nerveuse sympathique.

Les composés de l'invention peuvent donc être utilisés pour le traitement symptomatique des maladies et affections impliquant une hyperactivité du système $\alpha$-adrénergique au niveau du bas appareil urinaire, et notamment pour le traitement de l'hypertrophie bénigne de la prostate, de la dysurie, de la pollakiurie.

A cet effet ils peuvent être présentés sous toutes formes appropriées à l'administration entérale ou parentérale, associés à des excipients pharmaceutiques, par exemple sous forme de comprimés, dragées, gélules, capsules, solutions ou suspensions buvables ou injectables, suppositoires, étant dosés pour permettre une dose journalière de 0,5 à 500 mg de substance active.

**Revendications**

**Revendications pour les Etats contractants suivants : AT, BE, CH, DE, DK, FR, GB, IT, LI, LU, MC, NL, PT, SE**

1. Composé, sous forme d'énantiomère pur ou de mélange d'énantiomères, répondant à la formule générale (I)

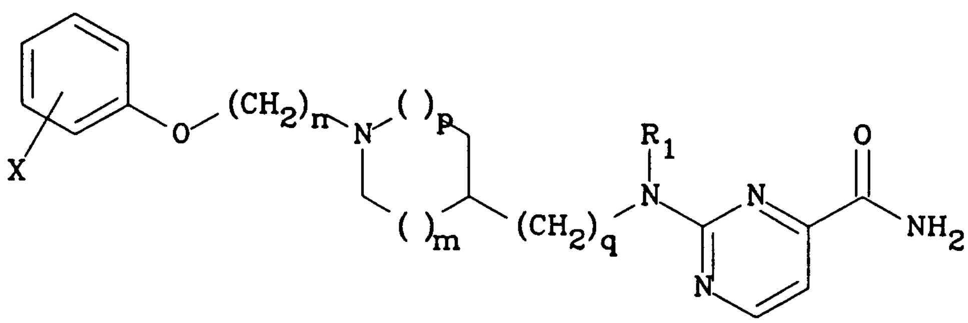

(I)

dans laquelle

X représente un ou plusieurs substituants choisis parmi les atomes d'hydrogène, de fluor et de chlore et les groupes méthyle, 1-méthyléthyle et méthoxy,
n représente le nombre 2 ou 3,
m représente le nombre 1, auquel cas p représente le nombre 1, ou bien
m représente le nombre 0, auquel cas p représente le nombre 2,
q représente le nombre 0 ou 1, et
$R_1$ représente un atome d'hydrogène,

à l'état de base ou de sel d'addition à un acide.

2. Procédé de préparation d'un composé selon la revendication 1, caractérisé en ce qu'on fait d'abord réagir une amine de formule générale (II)

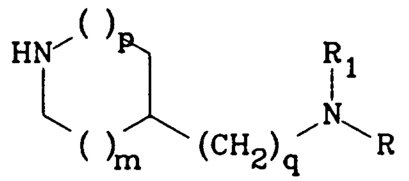

(II)

dans laquelle m, p, q et $R_1$ sont tels que définis dans la revendication 1 et R représente un groupement protecteur de l'amine, avec un halogénure de phénoxyalkyle de formule générale (III)

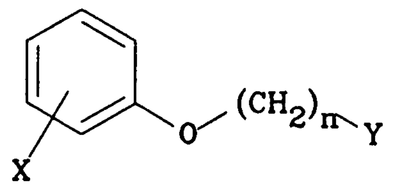

(III)

dans laquelle X et n sont tels que définis dans la revendication 1 et Y représente un atome de chlore ou de brome, dans un solvant aprotique et en présence d'une base, pour obtenir une diamine de formule générale (IV)

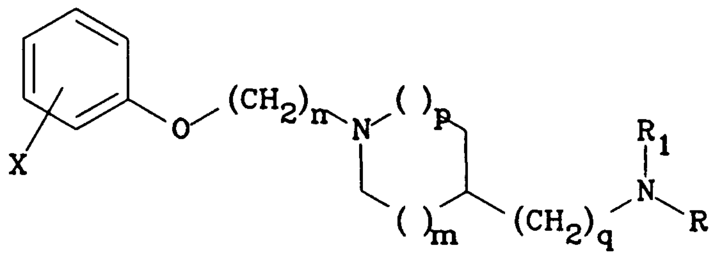

(IV)

que l'on déprotège ensuite pour obtenir une diamine de formule générale (V)

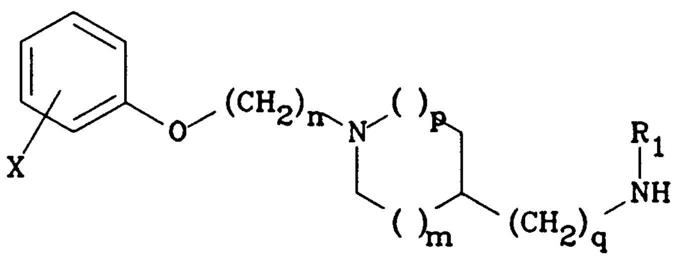

(V)

que l'on fait finalement réagir avec le 2-chloropyrimidine-4-carboxamide dans un solvant aprotique et en présence d'une base.

3. Médicament caractérisé en ce qu'il consiste en un composé selon la revendication 1.

4. Composition pharmaceutique caractérisée en ce qu'elle contient un composé selon la revendication 1, en association avec tout excipient approprié.

**Revendication pour les Etats contractants suivants : ES, GR**

**1.** Procédé de préparation d'un composé répondant à la formule générale (I)

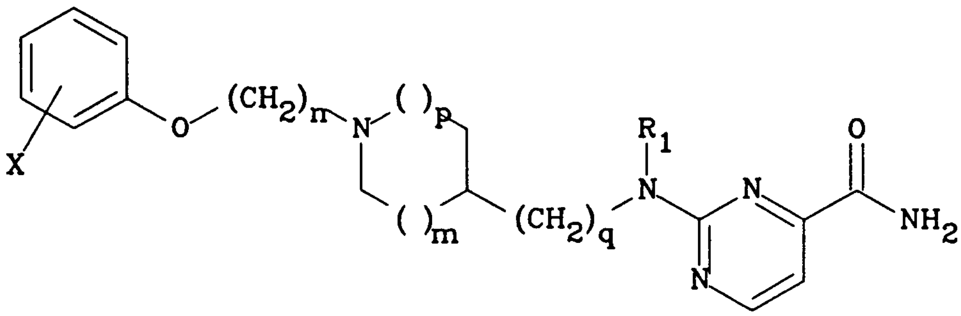

(I)

dans laquelle

- X représente un ou plusieurs substituants choisis parmi les atomes d'hydrogène, de fluor et de chlore et les groupes méthyle, 1-méthyléthyle et méthoxy,
- n représente le nombre 2 ou 3,
- m représente le nombre 1, auquel cas p représente le nombre 1, ou bien
- m représente le nombre 0, auquel cas p représente le nombre 2,
- q représente le nombre 0 ou 1, et
- $R_1$ représente un atome d'hydrogène,

procédé caractérisé en ce qu'on fait d'abord réagir une amine de formule générale (II)

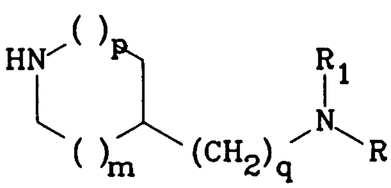

(II)

dans laquelle m, p, q et $R_1$ sont tels que définis ci-dessus et R représente un groupement protecteur de l'amine, avec un halogénure de phénoxyalkyle de formule générale (III)

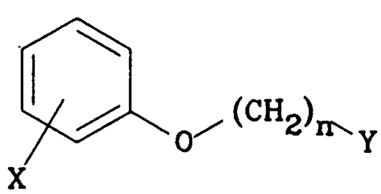

(III)

dans laquelle X et n sont tels que définis ci-dessus et Y représente un atome de chlore ou de brome, dans un solvant aprotique et en présence d'une base, pour obtenir une diamine de formule générale (IV)

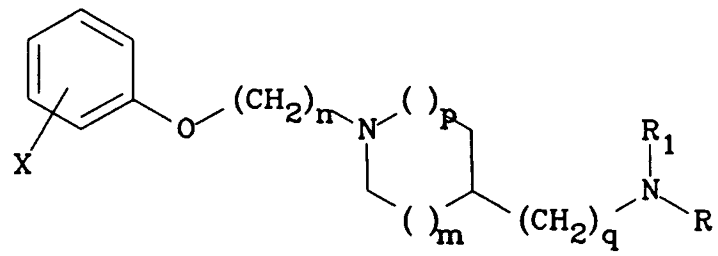

(IV)

que l'on déprotège ensuite pour obtenir une diamine de formule générale (V)

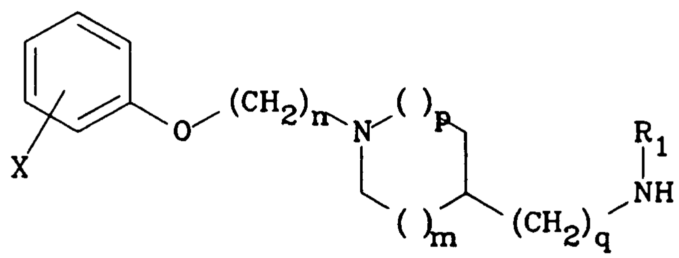

(V)

que l'on fait finalement réagir avec le 2-chloropyrimidine-4-carboxamide dans un solvant aprotique et en présence d'une base.

## Claims

**Claims for the following Contracting States : AT, BE, CH, DE, DK, FR, GB, IT, LI, LU, MC, NL, PT, SE**

**1.** Compound, in the form of a pure enantiomer or of a mixture of enantiomers, corresponding to the general formula (I)

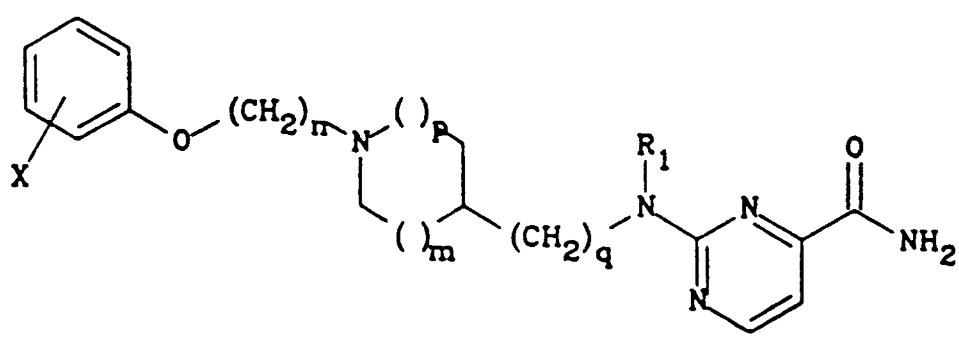

(I)

in which

- X represents one or more substituents chosen from hydrogen, fluorine and chlorine atoms and the methyl, 1-methylethyl and methoxy groups,
- n represents the number 2 or 3,
- m represents the number 1, in which case p represents the number 1, or else
- m represents the number 0, in which case p represents the number 2,
- q represents the number 0 or 1, and
- $R_1$ represents a hydrogen atom, in the base form or in the form of an addition salt with an acid.

**2.** Process for the preparation of a compound according to Claim 1, characterised in that an amine of general formula (II)

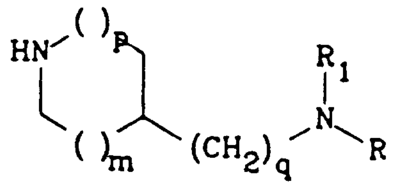

(II)

in which m, p, q and $R_1$ are as defined in Claim 1 and R represents an amine-protecting group, is first reacted with a phenoxyalkyl halide of general formula (III)

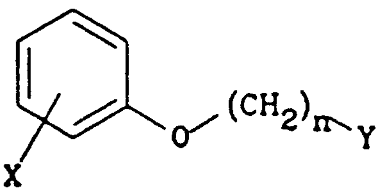

(III)

in which X and n are as defined in Claim 1 and Y represents a chlorine or bromine atom, in an aprotic solvent and in the presence of a base, in order to obtain a diamine of general formula (IV)

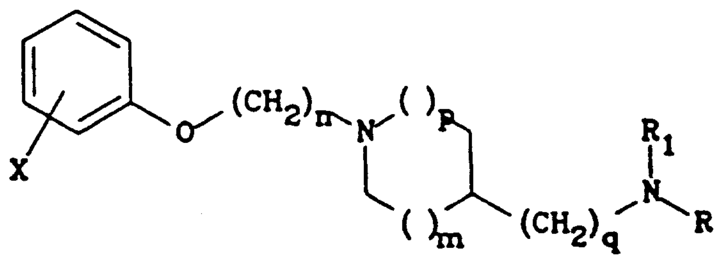

(IV)

which is then deprotected to obtain a diamine of general formula (V)

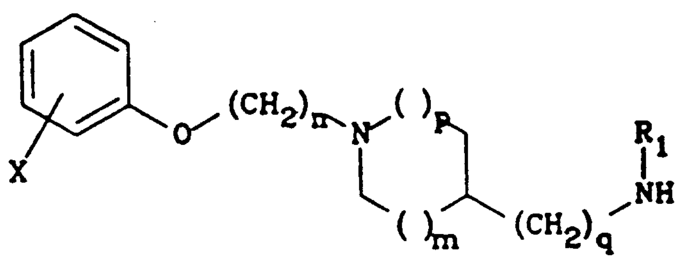

(V)

which is finally reacted with 2-chloropyrimidine-4-carboxamide in an aprotic solvent and in the presence of a base.

**3.** Medicament characterised in that it consists of a compound according to Claim 1.

**4.** Pharmaceutical composition, characterised in that it contains a compound according to Claim 1, in combination with any appropriate excipient.

**Claim for the following Contracting States : ES, GR**

**1.** Process for the preparation of a compound corresponding to the general formula (I)

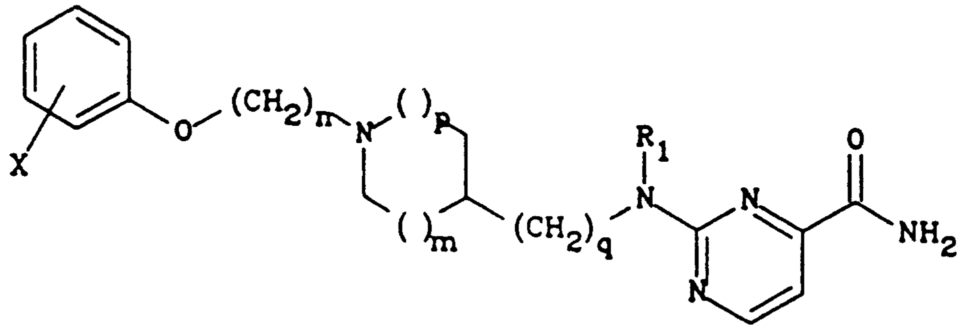

(I)

in which

X represents one or more substituents chosen from hydrogen, fluorine and chlorine atoms and the methyl, 1-methylethyl and methoxy groups,

n represents the number 2 or 3,

m represents the number 1, in which case p represents the number 1, or else

m represents the number 0, in which case p represents the number 2,

q represents the number 0 or 1, and

$R_1$ represents a hydrogen atom, which process is characterised in that an amine of general formula (II)

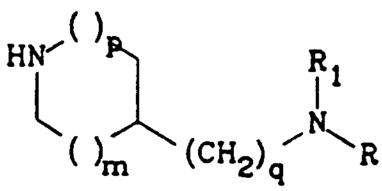

(II)

in which m, p, q and $R_1$ are as defined above and R represents an amine-protecting group, is first reacted with a phenoxyalkyl halide of general formula (III)

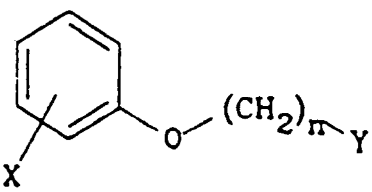

(III)

in which X and n are as defined above and Y represents a chlorine or bromine atom, in an aprotic solvent and in the presence of a base, in order to obtain a diamine of general formula (IV)

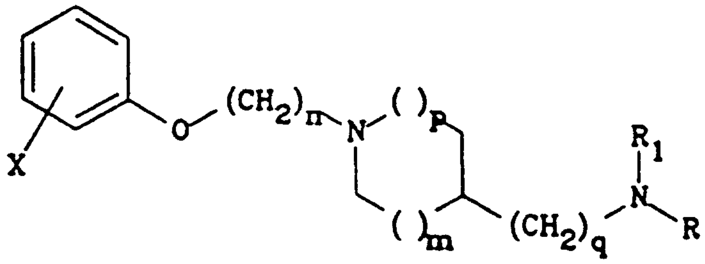

(IV)

which is then deprotected to obtain a diamine of general formula (V)

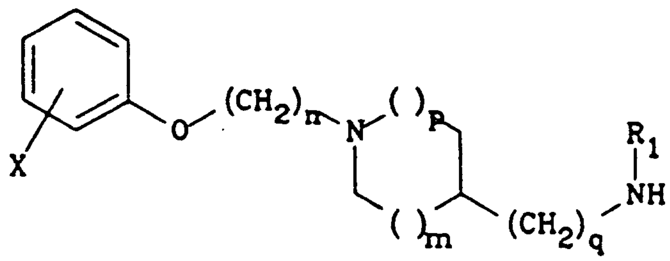

(V)

which is finally reacted with 2-chloropyrimidine-4-carboxamide in an aprotic solvent and in the presence of a base.

**Patentansprüche**

**Patentansprüche für folgende Vertragsstaaten : AT, BE, CH, DE, DK, FR, GB, IT, LI, LU, MC, NL, PT, SE**

1. Verbindung, die in Form des reinen Enantiomeren oder einer Mischung von Enantiomeren vorliegt, der allgemeinen Formel (I)

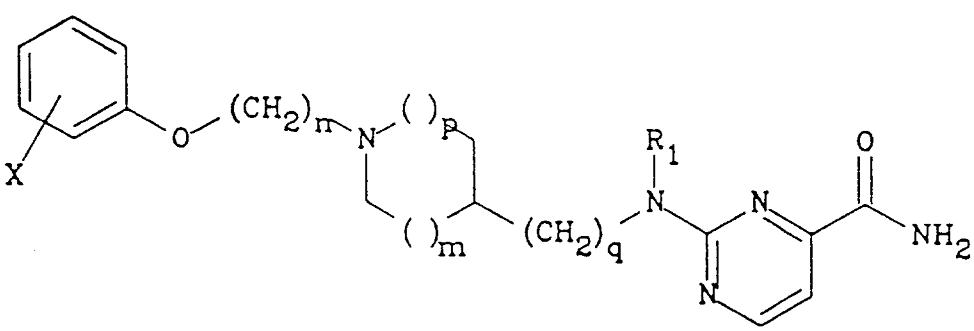

(I)

in der
- X einen oder mehrere Substituenten ausgewählt aus Wasserstoffatomen, Fluoratomen, Chloratomen, Methylgruppen, 1-Methylethylgruppen und Methoxygruppen,
- n die Zahl 2 oder 3,
- m die Zahl 1, und in diesem Fall p die Zahl 1, oder
- m die Zahl 0, und in diesem Fall p die Zahl 2,
- q die Zahl 0 oder 1 und
- $R_1$ ein Wasserstoffatom

bedeuten,
in Form der Base oder des Additionssalzes mit einer Säure.

**2.** Verfahren zur Herstellung einer Verbindung nach Anspruch 1, **dadurch gekennzeichnet**, daß man zunächst ein Amin der allgemeinen Formel (II)

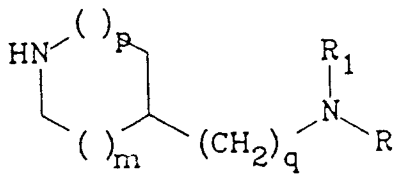

(II)

in der m, p, q und $R_1$ die in Anspruch 1 angegebenen Bedeutungen besitzen und R eine Schutzgruppe für das Amin darstellt, mit einem Phenoxyalkylhalogenid der allgemeinen Formel (III)

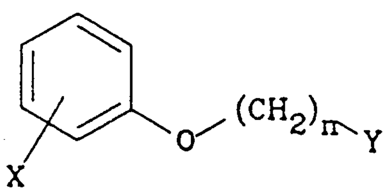

(III)

in der X und n die in Anspruch 1 angegebenen Bedeutungen besitzen und Y ein Chlor- oder Bromatom bedeutet, in einem aprotischen Lösungsmittel und in Gegenwart einer Base umsetzt zur Bildung eines Diamins der allgemeinen Formel (IV)

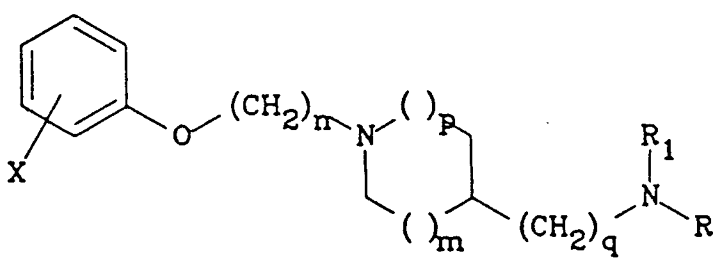

(IV)

von welcher man anschließend die Schutzgruppe abspaltet zur Bildung eines Diamins der allgemeinen Formel (V)

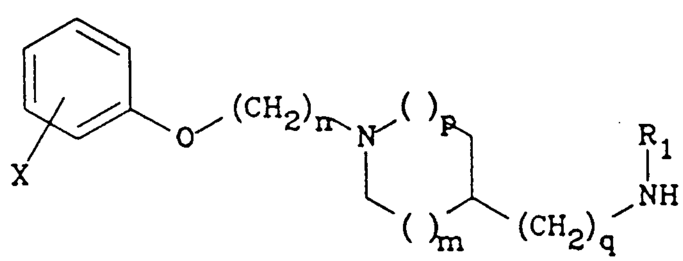

(V)

welches man schließlich in einem aprotischen Lösungsmittel und in Gegenwart einer Base mit 2-Chlorpyrimidin-4-carboxamid umsetzt.

**3.** Arzneimittel, **dadurch gekennzeichnet**, daß es aus einer Verbindung nach Anspruch 1 besteht.

**4.** Pharmazeutische Zubereitung, **dadurch gekennzeichnet**, daß sie eine Verbindung nach Anspruch 1 in Kombination mit irgendeinem geeigneten Trägermaterial enthält.

**Patentanspruch für folgende Vertragsstaaten : ES, GR**

1. Verfahren zur Herstellung einer Verbindung der allgemeinen Formel (I)

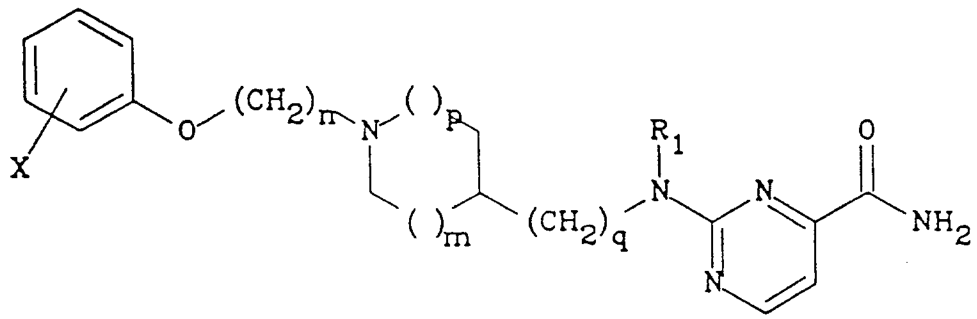

(I)

in der

| | |
|---|---|
| X | einen oder mehrere Substituenten ausgewählt aus Wasserstoffatomen, Fluoratomen, Chloratomen, Methylgruppen, 1-Methylethylgruppen und Methoxygruppen, |
| n | die Zahl 2 oder 3, |
| m | die Zahl 1, und in diesem Fall p die Zahl 1, oder |
| m | die Zahl 0, und in diesem Fall p die Zahl 2, |
| q | die Zahl 0 oder 1 und |
| $R_1$ | ein Wasserstoffatom |

bedeuten,

**dadurch gekennzeichnet**, daß man zunächst ein Amin der allgemeinen Formel (II)

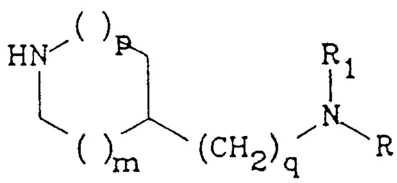

(II)

in der m, p, q und $R_1$ die oben angegebenen Bedeutungen besitzen und R eine Schutzgruppe für das Amin darstellt, mit einem Phenoxyalkylhalogenid der allgemeinen Formel (III)

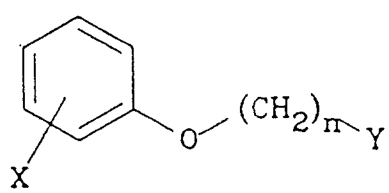

(III)

in der X und n die oben angegebenen Bedeutungen besitzen und Y ein Chlor- oder Bromatom bedeutet, in einem aprotischen Lösungsmittel und in Gegenwart einer Base umsetzt zur Bildung eines Diamins der allgemeinen Formel (IV)

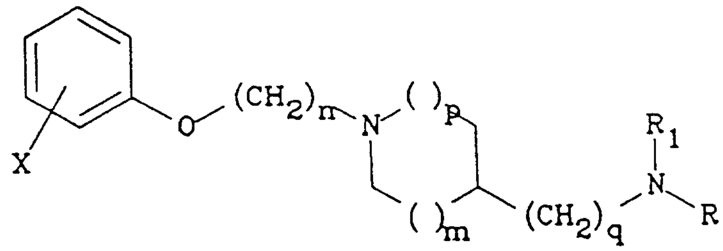

(IV)

von welcher man anschließend die Schutzgruppe abspaltet zur Bildung eines Diamins der allgemeinen Formel (V)

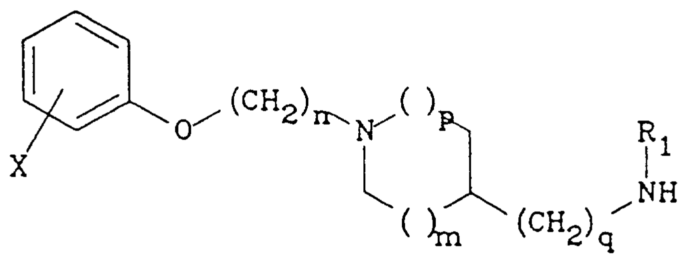

(V)

welches man schließlich in einem aprotischen Lösungsmittel und in Gegenwart einer Base mit 2-Chlorpyrimidin-4-carboxamid umsetzt.